Europäisches Patentamt

European Patent Office

Office européen des brevets

(11) Publication number: **0 367 509**
**A2**

# EUROPEAN PATENT APPLICATION

(21) Application number: **89311096.5**

(22) Date of filing: **27.10.89**

(51) Int. Cl.5 **A61K 31/425 , A61K 9/20 , A61K 9/48**

(30) Priority: **01.11.88 GB 8825541**

(43) Date of publication of application:
**09.05.90 Bulletin 90/19**

(34) Designated Contracting States:
**DE NL SE**

(71) Applicant: **FISONS plc**
**Fison House Princes Street**
**Ipswich Suffolk IP1 1QH(GB)**

(72) Inventor: **Tapley, Ian Robert**
**16 Highfields Close**
**Shepshed Leicestershire(GB)**

(74) Representative: **Wright, Robert Gordon McRae**
**FISONS plc 12 Derby Road**
**Loughborough Leicestershire LE11 0BB(GB)**

(54) **Formulations.**

(57) There are described new pharmaceutical formulations and methods for their preparation.

Formulations comprising 5-t-butyl-3-[N-(1-(S)-ethoxycarbonyl-3-phenylpropyl)-L-alanyl]-2,3-dihydro-1,3,4-thiadiazole-2-(S)-carboxylic acid, and pharmaceutically acceptable salts, esters, esters and amides thereof, as active ingredient in admixture with a pharmaceutically acceptable adjuvant, diluent or carrier in a form suitable for compression into a tablet or a capsule suitable for oral administration are described.

There is also provided a unit dose formulation comprising from 1µg to 1mg of the aforementioned active ingredient

Also described are processes for the manufacture of a pharmaceutical formulations.

EP 0 367 509 A2

## Formulations

This invention relates to new pharmaceutical formulations and methods for their preparation.

Known formulations comprising angiotensin converting enzyme (ACE) inhibitors generally comprise relatively high doses of the active ingredient. Such formulations suffer from the disadvantage that high doses of ACE inhibitors may cause prolonged undesirable side effects in patients, for example, renal failure or cough. ACE inhibitors also require careful prescribing to patients who require diabetic and/or renal control.

5-t-Butyl-3-[N-(1-(S)-ethoxycarbonyl-3-phenylpropyl)-L-alanyl]-2,3-dihydro-1,3,4-thiadiazole-2-(S)-carboxylic acid is described in European Patent Application No. 0217519. The aforementioned compound is useful, inter alia, as an ACE inhibitor.

Conventional formulations of ACE inhibitors comprise the active ingredient in admixture with a dry carrier. Such formulations if applied to low unit dose formulations suffer from the disadvantage that the distribution of the active ingredient may not be uniform, giving rise to the risk of an individual tablet or capsule comprising an amount of active ingredient below the required dosage level. Tablet and capsule formulations of 5-t-butyl-3-[N-(1-(S)-ethoxycarbonyl-3-phenylpropyl) -L-alanyl]-2,3-dihydro-1,3,4-thiadiazole-2-(S)-carboxylic acid containing relatively higher doses of the active ingredient are also novel.

However, we have now surprisingly found formulations, and particularly low unit dosage formulations, of 5-t-butyl-3-[N-(1-(S)-ethoxycarbonyl-3-phenylpropyl) -L-alanyl]-2,3-dihydro-1,3,4-thiadiazole-2-(S)-carboxylic acid which overcome or mitigate the disadvantages of known formulations.

According to the invention we provide a unit dose formulation comprising from 1µg to 1mg of 5-t-butyl-3-[N-(1-(S)-ethoxycarbonyl-3-phenylpropyl)-L-alanyl]-2,3-dihydro-1,3,4-thiadiazole-2-(S)-carboxylic acid, or a pharmaceutically acceptable salt, ester or amide thereof, as active ingredient in admixture with a pharmaceutically acceptable adjuvant, diluent or carrier.

The formulation according to the invention preferably comprises from 100µg to 1mg, more preferably from 250µg to 1mg and most preferably from 500µg to 1mg, eg. 750µg.

According to a further feature of the invention we provide a pharmaceutical formulation comprising 5-t-butyl-3-[N-(1-(S)-ethoxycarbonyl-3-phenylpropyl)-L-alanyl]-2,3-dihydro-1,3,4-thiadiazole-2-(S)-carboxylic acid, or a pharmaceutically acceptable salt, ester or amide thereof, in admixture with a pharmaceutically acceptable adjuvant, diluent or carrier in a form suitable for compression into a tablet suitable for oral administration.

Tablet formulations according to the invention may comprise from 1µg to 500mg, preferably from 1µg to 50mg, more preferably from 1µg to 10mg and most preferably from 1µg to 5mg eg 1 or 2mg of 5-t-butyl-3-[N-(1-(S)-ethoxycarbonyl-3-phenylpropyl)-L-alanyl]-2,3-dihydro-1,3,4-thiadiazole-2-(S)-carboxylic acid, or a pharmaceutically acceptable salt, ester or amide thereof, as active ingredient in admixture with a pharmaceutically acceptable adjuvant, diluent or carrier.

Tablet formulations according to this aspect of the invention may also provide a low unit dose of the active ingredient. Therefore we provide a tablet formulation as hereinbefore described comprising from 1µg to 1mg of active ingredient, preferably from 100µg to 1mg, more preferably 250µg to 1mg and most preferably 500µg to 1mg of active ingredient in admixture with a pharmaceutically acceptable adjuvant, diluent or carrier.

According to the invention we further provide a process for the manufacture of a pharmaceutical formulation according to the invention which comprises mixing the active ingredient with a pharmaceutically acceptable adjuvant, diluent or carrier.

Tablet formulations may be prepared as direct compression formulations or wet granulation formulations.

According to a further feature of the invention we provide a pharmaceutical formulation comprising 5-t-butyl-3-[N-(1)-(S)-ethoxycarbonyl-3-phenylpropyl)-L-alanyl]-2,3-dihydro-1,3,4-thiadiazole-2-(S)-carboxylic acid, or a pharmaceutically acceptable salt, ester or amide thereof, in admixture with a pharmaceutically acceptable adjuvant diluent or carrier in a form suitable for filling into a capsule suitable for oral administration.

The capsule formulation hereinbefore described may comprise from 1µg to 500mg, preferably from 1µg to 50mg, more preferably from 1µg to 10mg and most preferably from 1µg to 5mg, eg 1 or 2mg 5-t-butyl-3-[N-(1)-(S)-ethoxycarbonyl-3-phenylpropyl)-L-alanyl]-2,3-dihydro-1,3,4-thiadiazole-2-(S)-carboxylic acid, or a pharmaceutically acceptable salt, ester or amide thereof, as active ingredient in admixture with a pharmaceutically acceptable adjuvant diluent or carrier.

Capsule formulations according to the invention may provide a low unit dose of the active ingredient.

Therefore we provide a capsule formulation as hereinbefore described comprising from 1μg to 1mg of active ingredient. preferably from 100μg to 1mg, more preferably from 250μg to 1mg and most preferably 500μg to 1mg, eg 750μg, of active ingredient in admixture with a pharmaceutically acceptable adjuvant diluent or carrier.

The capsule formulation according to the invention may comprise the active ingredient dispersed in an inert carrier wherein at least 90% w/w of the active ingredient is present as a monomolecular dispersion within a thermosetting or thixotropic vehicle, which vehicle may be hydrophobic or hydrophilic.

We prefer a formulation in which at least 95% w/w, preferably at least 96% w/w and more preferably 97% w/w of the active ingredient is present as a monomolecular dispersion within a thermosetting or thixotropic vehicle as hereinbefore described.

We further provide a process for the manufacture of a capsule formulation according to the invention which comprises dissolving the active ingredient in a molten thermosetting or thixotropic vehicle.

Inert vehicles which may be used include polyalkylene glycols having a mean molecular weight of, eg from 1000 to 8000. Such polyalkyleneglycols include polypropyleneglycols having a mean molecular weight of, eg from 1000 to 8000; and preferably polyethyleneglycols (PEG) having a mean molecular weight of from 1000 to 8000, eg PEG 1000 to PEG 8000 such as PEG 1500, PEG 4000, PEG 6000, PEG 8000 and especially PEG 1000; polyglycols; LUTROL 9; CARBOWAXES; inert vehicles available under the name GELUCIRE; natural or hardened oils, waxes, etc; emulsifying bases, eg 'Witepsol' bases, consisting of hydrogenated triglycerides of lauric acid with added monoglycerides; 'Massupol' bases, which consist of glyceryl esters of lauric acid with a very small amount of glyceryl monostearate; colloidal silicates, eg silicon dioxide; lower alcohols having 2 to 8 carbon atoms and 1 to 3 hydroxy groups, eg glycerol, propyleneglycol, butyleneglycol or benzyl alcohol. (GELUCIRE, LUTROL and CARBOWAX are Registered Trade Marks).

We further provide a formulation as hereinbefore described in which the particle size of a substantial amount of the active ingredient is less than 53μm diameter, preferably less than 25μm diameter and more preferably less than 10μm diameter, most preferably less than 6μm diameter, eg. less than 5μm. The particle size of a substantial amount of the active ingredient may be greater than 1μm diameter, preferably greater than 2μm diameter, more preferably 2.5μm diameter and most preferably 3μm diameter. The preferred particle size of a substantial amount of the active ingredient is from 3 to 5μm diameter. The particle size of the adjuvants and excipients may be varied according to the formulation desired.

By the term a substantial amount of the active ingredient we mean greater than 50%w/w of the active ingredient, preferably greater than 60%w/w, more preferably greater than 70%w/w, most preferably greater than 80%w/w, and especially greater than 90%w/w, eg. greater than 95%w/w.

Pharmaceutically acceptable salts of 5-t-butyl-3-[N-(1-(S)-ethoxycarbonyl-3-phenylpropyl)-L-alanyl]-2,3-dihydro-1,3,4-thiadiazole-2-(S)-carboxylic acid include ammonium salts, alkali metal salts, eg. sodium and potassium salts; alkaline earth metal salts, eg. the calcium and magnesium salts; salts with organic bases, eg. salts with dicyclohexylamine or N-methyl-D-glucamine; and salts with amino acids, eg. with arginine, lysine, etc. Also, when the molecule contains a basic group, salts with organic or inorganic acids, eg. with HCl, HBr, $H_2SO_4$, $H_2PO_4$, methanesulphonic, toluene sulphonic, maleic, fumaric or camphorsulphonic acids. The non-toxic physiologically acceptable salts are preferred, although other salts are also useful, eg. in isolating or purifying the product.

Pharmaceutically acceptable esters include esters with C1 to 10 alcohols, eg. alkyl C 1 to 6 esters and esters with benzyl alcohol. The amides may be, for example, unsubstituted or mono-or di-C 1 to 6 alkyl amides and may be made by conventional techniques, eg. reaction of an ester of the corresponding acid with ammonia or an appropriate amine.

The formulations of the invention are advantageous in that they may be more efficaceous, produce less side effects, are longer acting, more readily absorbed, less toxic, distributed in the body tissues in a different manner, more selective in their action, excreted in a different manner or have other advantageous properties when compared to other similar formulations.

The formulations of the invention are useful because they possess pharmacological properties. In particular they inhibit angiotensin converting enzyme and thus block conversion of the decapeptide angiotensin I to angiotensin II (see Example A). Angiotensin II is a potent vasoconstrictor in mammals. It also stimulates aldosterone release which results in salt and fluid retention. Increased blood pressure is the physiological result of these changes. Inhibitors of angiotensin converting enzyme are thus effective antihypertensive agents in a variety of animal models and are indicated for use clinically (see Example B), for example in patients with renovasular, malignant or essential hypertension or chronic congestive heart failure. See, for example, D W Cushman et al., Biochemistry 16, 5484 (1977) and E W Petrillo and MA Ondetti, Med. Res. Rev. 2 93 (1982).

Thus, the formulations of this invention are useful as antihypertensives in treating hypertensive mammals, including humans and they can be utilised to achieve reduction of blood pressure, or in the treatment or alleviation of myocardial hypertrophy, ischaemia or arrhythmia, eg in formulations containing appropriate pharmaceutically acceptable excipients, diluents or carriers. The formulations of the invention can be administered (to animals or humans) in unit dosages of 1 to 500mg generally given several times, eg 1 to 4 times, per day thus giving a total daily dose of from 1 to 2000mg per day.The low unit dosages of the present invention can be administered in dosages of 1μg to 1mg generally given several times, eg 1 to 4 times, per day thus giving a total daily dose of from 1μg to 4mg per day. A unit dosage of from 100μg to 1mg is preferred, more preferably from 250μg to 1mg and most preferably from 500μg to 1mg.

The aforementioned doses will vary depending on the type and severity of disease, weight of patient and other factors which a person skilled in the art will recognise.

According to a further feature of the invention we provide the use of 5-t-butyl-3-[N-1-(S)-ethoxycarbonyl-3-phenylpropyl)-L-alanyl]-2,3-dihydro-1,3,4-thiadiazole-2-(S)-carboxylic acid, or a pharmaceutically acceptable salt, ester or amide thereof, in the manufacture of a medicament for the treatment or alleviation of hypertension.

We further provide a method of treatment of hypertension which comprises administering a therapeutically effective amount of a formulation as hereinbefore described to a patient suffering from such a condition.

There is also provided a pharmaceutical formulation as hereinbefore described comprising up to 5% w/w of 5-t-butyl-3-[N-1-(S)-ethoxycarbonyl-3-phenylpropyl)-L-alanyl]-2,3-dihydro-1,3,4-thiadiazole-2-(S)-carboxylic acid, or a pharmaceutically acceptable salt, ester or amide thereof, as active ingredient in admixture with a pharmaceutically acceptable adjuvant, diluent or carrier preferably up to 2% w/w, more preferably up to 1% w/w, eg. 0.25% w/w or 0.5% w/w.

The low unit dosage formulations of the invention may be administered by a wide variety of routes and may act systemically or locally. Thus the formulations may be administered by oral or nasal inhalation to the lung, directly to the nose or eye, to the buccal cavity, oesophageally, rectally, topically to the skin or to other available surfaces of the body, by instillation into the bladder, by injection, eg. intravenously, intramuscularly, intraperitoneally, or by surgical implant. The formulations may by administered directly to the organ or part of the body showing symptoms or to a part remote from that showing symptoms. Thus skin conditions may be treated by direct application to the area affected, or by systemic, eg. oesophageal, administration.

Thus the formulations may be formulated in a manner suitable for application to the skin of the animal, eg. as an ointment, as a cream, which may be either an oil in water type, or a water in oil type, as a lotion or liniment, as a paste or gel. A semi-solid base that may be mentioned comprises fatty alcohol/glycol mixture.

When the formulations are to be used in aqueous solution we prefer the solution to be clear and to this end it may be necessary to make the solution with very pure water, eg. containing very low amounts of dibasic, e.g. magnesium or calcium, ions, or to incorporate a chelating or sequestering agent in the solution.

The formulations may be formulated as a dentifrice composition, eg. a toothpaste of a toothpowder, which may contain, for example an abrasive, a detergent and/or a humectant.

When the formulations are to be used to treat the eye they may be used, for example, in the form of an aqueous solution, or an ophthalmic ointment (eg. in an oily base) or in a controlled release formulation, eg. a device adapted to be inserted under the eyelid and to release the new compound at a controlled rate.

For oral or rectal administration the formulations may be worked up with inorganic or organic pharmaceutically acceptable adjuvants or excipients. Examples of such adjuvants are:

For tablets, lozenges and dragees: Binders, for example, povidone or cellulosic materials, eg. microcrystalline cellulose and methyl cellulose; disintegrating agents, for example starches, eg. maize starch; stabilisers, eg. against hydrolysis of the active ingredients; flavouring agents, for example sugars such as lactose; fillers; stearates and inorganic lubricants, eg. talc.

For syrups, suspensions, emulsions or dispersions: A liquid vehicle in which the active ingredients may be dissolved or suspended, e.g. water; and suspending agents, e.g. cellulose derivaties, gums etc.

For hard or soft capsules: Diluents, e.g. lactose; glidants, e.g. stearates; inorganic materials, e.g. silica or talc; stabilisers and dispersing agents.

For suppositories; Natural or hardened oils, waxes etc. A large number of proprietary emulsifying bases are available and are suitable for use in suppositories. These include 'Witepsol' bases, consisting of hydrogenated triglycerides or lauric acid with added monoglycerides; and 'Massupol' bases, which consist of glyceryl esters of lauric acid with a very small amount of glycerol monostearate.

For enemas: Water, sodium chloride, buffers etc, and optionally foam forming agents.

4

The abovementioned adjuvants and excipients may be used in any of the tablet and capsule formulations hereinbefore described.

The formulations may also contain further adjuvants, for example a formulation for use in tablet form may contain flow aids and glidants to assist in tabletting, e.g. magnesium stearate or colloidal silica; or wetting agents to assist in granulation, e.g. dioctyl sodium sulphosuccinate. The formulation may also if desired contain a pharmaceutically acceptable dye or colourant, and may, if desired, be coated using conventional film or sugar coating techniques.

For direct compression tablet formulations the preferred adjuvants or excipients include maize starch, eg. 0-5% w.w; dibasic calcium phosphate, eg. 0-50% w/w; microcrystalline cellulose, eg. 50-90% w/w; sodium starch glycolate, eg. 2-10% w/w; and magnesium stearate, eg. 1-4% w/w.

For wet granulation formulations the preferred adjuvants or excipients include maize starch, eg. 0-5% w/w; dibasic calcium phosphate, eg. 0-50% w/w; microcrystalline cellulose, eg. 50-90% w/w; sodium starch glycolate, eg. 2-10% w/w; magnesium stearate, eg. 1-4% w/w; povidone, eg. 0.5-3% w/w and water as required.

If desired the formulations may be formulated in sustained release form, e.g. by coating the drug particles with a layer of a substance which could be expected to be slowly dissolved or digested or to act as semi-permeable membranes throught which drug can diffuse when the preparations are ingested. Specifically there may be mentioned enteric coated formulations.

For administration by inhalation the formulations may be formulated with a compressed gas, e.g. nitrogen, or a liquified propellant as a pressurised aerosol composition.

The formulations of the invention may be used in a variety of dosing schedules, as hereinbefore described, either on their own or in conjunction with one or more of the other active ingredients listed herein. Thus a priming dose of the formulation of the present invention may be followed by a maintenance dose of the same or another formulation. The priming dose may be substantially smaller or substantially larger than the maintenance dose. The new formulations when used in conjunction with another active ingredient may be used together with, before or after the other active ingredient depending on the desired combined effect of the formulation. The different active agents may be administered by the same or different routes.

The other pharmaceutically active compounds may be, for example, diuretics of antihypertensives. The dosage of the other pharmaceutically active compound can be that conventionally used when the compound is administered on its own, but is preferably somewhat lower. To illustrate these combinations, the formulations of this invention effective clinically in the range, eg 1µg to 500mg per day, can be combined at levels ranging, eg from 1-200 milligrams per day with the following antihypertensives and diuretics in dose ranges per day as indicated:

hydrochlorothiazide (15-200mg), chlorthiazide (125-2000mg), ethacrynic acid (15-200mg), amiloride (5-20mg), furosemide (5-80mg), propanolol (20-480mg), timolol (5-50ml), nifedipine (20-100mg), verapamil (120-480mg) and methyldopa (65-2000mg). In addition, the triple drug combinations of hydrochlorothiazide (15-200mg) plus amiloride (5-20mg) plus converting enzyme inhibitor of the invention (1-200mg) or hydrochlorothiazide (15-200mg) plus timolol (5-50mg), plus the formulation of this invention 1µg to 5mg are contemplated. The above dose ranges may be adjusted on a unit bases as necessary to permit divided daily dosage. Also, the dose may vary depending on the severity of the disease, weight of patient and other factors which a person skilled in the art will recognise.

The invention will now be illustrated, but in no way limited by the following Example, in which temperatures are in degrees celsius.

Example 1

Capsule Formulation

Polyethyleneglycol with a mean molecular weight of 1000 (PEG 1000) and 5-t-butyl-3-[N-(1-(S)-ethoxycarbonyl-3-phenylpropyl)-L-alanyl]-2,3-dihydro-1,3,4-thiadiazole-2-(S)-carboxylic acid (active ingredient) were mixed together to produce a 0.25% w/w mixture of active ingredient to PEG 1000. The mixture was heated to 60° and 100mg portions were filled into gelatin capsules. The mixture was then allowed to cool producing capsules comprising 250µg of active ingredient.

Example 2

Tablet Formulations

| i) Direct Compression Formulation | |
|---|---|
| Constituent | Input per Tablet |
| Active ingredient | 0.25 mg |
| Maize Starch | 1.00 mg |
| Dibasic Calcium Phosphate | 30.00 mg |
| Microcrystalline Cellulose | 62.45 mg |
| Sodium Starch Glycolate | 5.00 mg |
| Magnesium Stearate | 1.30 mg |

| ii) Wet Granulation Formulation | |
|---|---|
| Constituent | Input per Tablet |
| Active ingredient | 0.25 mg |
| Maize Starch | 2.25 mg |
| Dibasic Calcium Phosphate | 30.00 mg |
| Microcrystalline Cellulose | 59.70 mg |
| Sodium Starch Glycolate | 5.00 mg |
| Magnesium Stearate | 1.30 mg |
| Povidone | 1.50 mg |
| Water (Purified) | 30.00 mg |

| iii) Direct Compression Formulation | |
|---|---|
| Constituent | Input per Tablet |
| Active ingredient | 1.00 mg |
| Maize Starch | 1.00 mg |
| Dibasic Calcium Phosphate | 30.00 mg |
| Microcrystalline Cellulose | 61.70 mg |
| Sodium Starch Glycolate | 5.00 mg |
| Magnesium Stearate | 1.30 mg |

| iv) Wet Granulation Formulation | |
|---|---|
| Constituent | Input per Tablet |
| Active ingredient | 2.00 mg |
| Maize Starch | 2.25 mg |
| Dibasic Calcium Phosphate | 30.00 mg |
| Microcrystalline Cellulose | 57.95 mg |
| Sodium Starch Glycolate | 5.00 mg |
| Magnesium Stearate | 1.30 mg |
| Povidone | 1.50 mg |
| Water (Purified) | 30.00 mg |

## Example A

In vitro Assay of inhibitors of Angiotensin Converting Enzyme.

The method is based upon that of Cushman and Cheung (1971) but uses a radioactive substrate [glycine-1$^{14}$C] hippuryl-L-histidyl-L-leucine (HHL) whose hydrolysis may be determined by liquid scintillation counting of released [$^{14}$C]-hippuric acid. Hydrolysis of 2mM HHL by an extract of rabbit lung acetone powder (Sigma) over a 30 minute incubation period at 37° is followed by acidification of the reaction mixture and extraction of [$^{14}$C]-hippurate with ethyl acetate.

Potential inhibitors are tested initally at 0.01mM and if found are retested at lower concentrations to determine an $IC_{50}$. Dimethyl sulphoxide at 1% final concentration may be used as a solubility aid without affecting enzyme activity. Compounds of special interest are studied at a range of substrate and inhibitor concentrations to determine the type of inhibition and are also tested against other enzymes, eg carboxypeptidase A to establish their specifity for ACE.

## Example B

Antyhypertensive effects were investigated in conscious spontaneously hypertensive rats (SHR) of the Okamoto strain. Systolic blood pressure and heart rate were measured by the tail cuff method using an electrosphygomomanometer 1 hour before and 1, 3, 5 and 24 hours after oral dosing with the compound (dose range 0.1-100 mg/kp p.o.). Percentage changes in each parameter were measured with respect to pretreatment control values.

## Example C

Single oral doses of 5-t-butyl-3-[N-(1-(S)-ethoxycarbonyl-3-phenylpropyl)-L-alanyl]-2,3-dihydro-1,3,4-thiadiazole-2-(S)-carboxylic acid were administered to groups of eight healthy male volunteers over the dose range 10μg to 1mg. There were seven dose increments in ascending order of magnitude, namely 10μg, 20μg, 50μg, 100μg, 250μg, 500μg, and 1.0mg. Within each group, five subjects were randomly selected to receive active compound, the remaining three received placebo control.

Safety, tolerability and pharmacodynamic data (blood pressure, pulse rate, electro-cardiogram) were collected on all volunteers, and plasma angiotensin converting enzyme (ACE) levels were assessed for 72 hours after doses of 250μg and above. At the lower doses (10μg to 100μg), samples will be taken at 0, 2 and 24 hours for ACE assay. Plasma samples for analysis of 5-t-butyl-3-[N-(1-(S)-ethoxycarbonyl-3-phenylpropyl)-L-alany 1]-2,3-dihydro-1,3,4-thiadiazole-2-(S)-carboxylic acid and its metabolite were collected from subjects receiving 250μg dose levels or greater for 48 hours after dosing.

Blood samples were collected at the following times: predose, 0.25, 0.5, 0.75, 1.0, 1.5, 2, 4, 6, 8, 24, and 48 hours.

Each sample was centrifuged and the supernatant placed in plastic tubes, frozen and stored at -20° awaiting assay.

## Claims

1. A unit dosage formulation comprising from 1µg to 1 mg of 5-t-butyl-3-[N-1-(S)-ethoxycarbonyl-3-phenylpropyl)-alanyl-2,3-dihydro-1,3,4-thiadiazole-2-(S)-carboxylic acid, or a pharmaceutically acceptable salt, ester or amide thereof, as active ingredient in admixture with a pharmaceutically acceptable carrier.

2. A pharmaceutical formulation comprising 5-t-butyl-3-[N-(1-(S)-ethoxycarbonyl-3-phenylpropyl)-L-alanyl]-2,3-dihydro-1,3,4-thiadiazole-2-(S)-carboxylic acid, or a pharmaceutically acceptable salt, ester or amide thereof, in admixture with a pharmaceutically acceptable adjuvant, diluent or carrier in a form suitable for compression into a tablet suitable for oral administration.

3. A formulation according to claim 1 comprising from 100µg to 1mg of active ingredient.

4. A formulation according to claims 1 or 2 in which the particle size of a substantial amount of the active ingredient is less than 53µm diameter.

5. A formulation according to claims 1 or 2 in which the particle size is greater than 1µm diameter.

6. A pharmaceutical formulation comprising 5-t-butyl-3-[N-(1)-(S)-ethoxycarbonyl-3-phenylpropyl)-L-alanyl]-2,3-dihydro-1,3,4-thiadiazole-2-(S)-carboxylic acid, or a pharmaceutically acceptable salt, ester or amide thereof, in admixture with a pharmaceutically acceptable adjuvant, diluent or carrier in a form suitable for filling into a capsule suitable for oral administration.

7. A formulation according to claim 6 which comprises the active ingredient dispersed in an inert carrier wherein at least 90% w/w of the active ingredient is present as a monomolecular dispersion within a thermosetting or thixotropic vehicle, which vehicle may be hydrophobic or hydrophilic.

8. A formulation according to claims 1, 2 or 6 comprising up to 5% w/w of active ingredient.

9. The use of a formulation according to claims 1, 2 or 6 in the manufacture of a pharmaceutical for the treatment of hypertension.

10. A process for the preparation of a formulation according to claim 1, 2 or 6 which comprises mixing 5-t-butyl-3-[N-1-(S)-ethoxycarbonyl-3-phenylpropyl)-L-alanyl]-2,3-dihydro-1,3,4-thiadiazole-2-(S)-carboxylic acid, or a pharmaceutically acceptable salt, ester or amide thereof, with a pharmaceutically acceptable adjuvant, diluent or carrier.